# EUROPEAN PATENT SPECIFICATION

(11) **EP 4 194 850 B1**
(45) Date of publication and mention of the grant of the patent: **21.01.2026**
(21) Application number: 21877364.6
(22) Date of filing: 22.09.2021
(51) Int. Cl.: G01N 31/00, G01N 1/00, G01N 1/10, G01N 33/00, G01N 33/18, G01N 21/77, G01N 21/78, G01N 1/40, G01N 21/94, G01N 31/22, G01N 35/00, G01N 35/10

(54) **MANAGEMENT SYSTEM FOR A WASTEWATER TREATMENT FACILITY.**
MANAGEMENTSYSTEM FÜR EINE ABWASSERAUFBEREITUNGSANLAGE.
SYSTÈME DE GESTION POUR UNE INSTALLATION DE TRAITEMENT DES EAUX USÉES.

(30) Priority: 09.10.2020 JP 2020170955
(43) Date of publication of application: 14.06.2023
(73) Proprietor: MITSUBISHI HEAVY INDUSTRIES, LTD., Chiyoda-ku Tokyo 100-8332 (JP)
(72) Inventor: CHIYOMARU Masaru, Tokyo 100-8332 (JP); KISHIMOTO Shinya, Tokyo 100-8332 (JP); TSUJIUCHI Tatsuya, Tokyo 100-8332 (JP); HIRATA Takuya, Tokyo 100-8332 (JP)
(74) Representative: Studio Torta S.p.A.
(86) International application number: PCT/JP2021/034803
(87) International publication number: WO 2022/075063

(56) References cited:
- EP-A2- 0 469 772
- WO-A1-2007/105322
- JP-A- 2000 321 263
- JP-A- 2003 053 374
- JP-A- 2007 309 835
- JP-A- 2009 288 021
- JP-A- 2010 137 115
- JP-A- 2012 223 690
- JP-A- 2012 239 947
- JP-A- 2017 136 571
- JP-A- 2017 207 377
- JP-A- 2019 081 151
- JP-A- H03 242 549
- JP-A- H03 258 385
- JP-A- H05 264 538
- JP-A- H06 194 314
- JP-A- H07 198 695
- JP-U- H0 559 287
- PUROLITE: "Unexpectedly unknown ● Characteristics and application fields of chelated resins", PUROLITE AN ECOLAB COMPANY, 9 September 2016 (2016-09-09), pages 1 - 4, XP055920662, Retrieved from the Internet <URL:http://www.purolite.co.jp/2016-09-09-2487> [retrieved on 20220512]

## Description

### Technical Field

The present invention relates to a wastewater treatment facility and to a method for managing a wastewater facility.

### Background Art

For example, in a carbon dioxide recovery apparatus, carbon dioxide in discharge water and exhaust gas is absorbed in an organic aqueous solution to be removed. The iron concentration in this absorbing liquid needs to be managed, and thus is analyzed manually on a regular basis (for example, on a weekly basis).

Patent Document 1 discloses removing of a suspension containing iron clad in a filter apparatus installed in a condensate system for a power plant.

### Citation List

### Patent Literature

Patent Document 1: Japanese Patent Application Laid-Open No. 61-181506
Patent Document 2: JP 2019 081151A

### Summary of Invention

### Technical Problem

For example, by checking the iron concentration in the absorbing liquid, it may be possible to find whether rust exists, rust is increasing, and the like. The monitoring of the iron concentration in a solution is preferably performed not only for the absorbing liquid, but also in an apparatus (for example, a wastewater treatment facility) that adds the iron component. In view of this, there has been a demand for accurate detection of the iron concentration in a solution.

The present invention was made in view of the above circumstances, and an object of the present disclosure is to provide a management system and a method for managing a wastewater facility. The wastewater management system of the present invention is defined in claim 1. The method of the present invention is defined in claim 10.

### Solution to Problem

A first aspect of the present disclosure is an analysis system including: a collection unit configured to collect soluble iron contained in a sample; a reaction unit configured to produce a reaction solution; a detection unit configured to detect an absorbance of the reaction solution; and a supply control unit configured to supply the soluble iron collected in the collection unit and a reagent to the reaction unit.

A second aspect of the present disclosure is an analysis method including: collecting soluble iron contained in a sample; producing a reaction solution using the soluble iron and a reagent; and detecting an absorbance of the reaction solution.

A third aspect of the present disclosure is an analysis program causing a computer to execute: collecting soluble iron contained in a sample; producing a reaction solution using the soluble iron and a reagent; and detecting an absorbance of the reaction solution.

### Advantageous Effects of Invention

The present disclosure provides an effect of enabling accurate analysis of a sample containing soluble iron.

### Brief Description of Drawings

FIG. 1 is a diagram illustrating an example of a configuration of a wastewater treatment facility according to a first embodiment of the present disclosure.
FIG. 2 is a diagram illustrating a schematic configuration of an analysis system according to the first embodiment of the present disclosure.
FIG. 3 is a diagram illustrating an example of a hardware configuration of a control device according to the first embodiment of the present disclosure.
FIG. 4 is a functional block diagram illustrating functions of a control device according to the first embodiment of the present disclosure.
FIG. 5 is a diagram illustrating an example of a relationship between absorbance and iron concentration according to the first embodiment of the present disclosure.
FIG. 6 is a flowchart illustrating an example of a procedure of analysis processing according to the first embodiment of the present disclosure.
FIG. 7 is a flowchart illustrating an example of a procedure of the analysis processing according to the first embodiment of the present disclosure.
FIG. 8 is a flowchart illustrating an example of a procedure of the analysis processing according to the first embodiment of the present disclosure.
FIG. 9 is a flowchart illustrating an example of a procedure of washing processing according to the first embodiment of the present disclosure.
FIG. 10 is a diagram illustrating an example of a configuration of a carbon dioxide recovery facility according to the first embodiment of the present disclosure.
FIG. 11 is a diagram illustrating a schematic configuration of a management system according to a second embodiment of the present disclosure.

### Description of Embodiments

### First Embodiment

A first embodiment of an analysis system and a management system, an analysis method, and an analysis program according to the present disclosure will be described below with reference to the drawings. In the present embodiment, a description will be given on an example of a case where an analysis system 40 is applied to a wastewater treatment facility 120. However, the analysis system 40 is not limited to the wastewater treatment facility 120, and can be applied to various apparatuses.

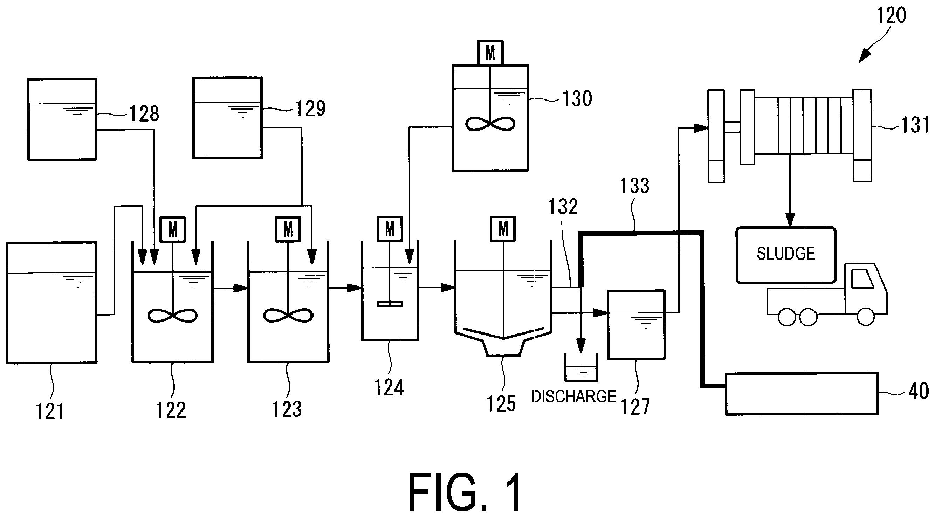

FIG. 1 is a diagram illustrating an example of a configuration of the wastewater treatment facility 120. A discharge water tank 121 stores discharge water from a desulfurization apparatus for example. The discharge water in the discharge water tank 121 is supplied to a first neutralization tank 122. In the first neutralization tank 122, the discharge water is mixed with acid from an acid storage 128 and caustic soda from a caustic soda storage 129. Then, the discharge water is supplied to a second neutralization tank 123 from the first neutralization tank 122, to be mixed with the caustic soda from the caustic soda storage 129. In this manner, pH adjustment is performed on the discharge water.

The discharge water is supplied from the second neutralization tank 123 to a floc tank 124. In the floc tank 124, the discharge water is mixed with flocculant (iron chloride for example) supplied from a flocculant dissolution storage 130. As a result, floc is formed. Then, from a floc sedimentation tank 125, a solid part is recovered as a sludge through a sludge tank 127 and a filter apparatus 131. On the other hand, a liquid part is discharged through a discharge line 132.

As described above, in the wastewater treatment facility 120, iron chloride is added to the discharge water. Since the iron chloride is dissolved in the discharge water, the discharge water discharged also includes an iron component. In the present embodiment, an example of a case is described where the discharged water discharged is a liquid analysis target (hereinafter referred to as "sample"). The analysis target may be any liquid containing an iron component, and is not limited to the one described above.

Thus, as illustrated in FIG. 1, the analysis system 40 is applied to the discharge line 132 on the outlet side of the floc sedimentation tank 125 through a provision line 133.

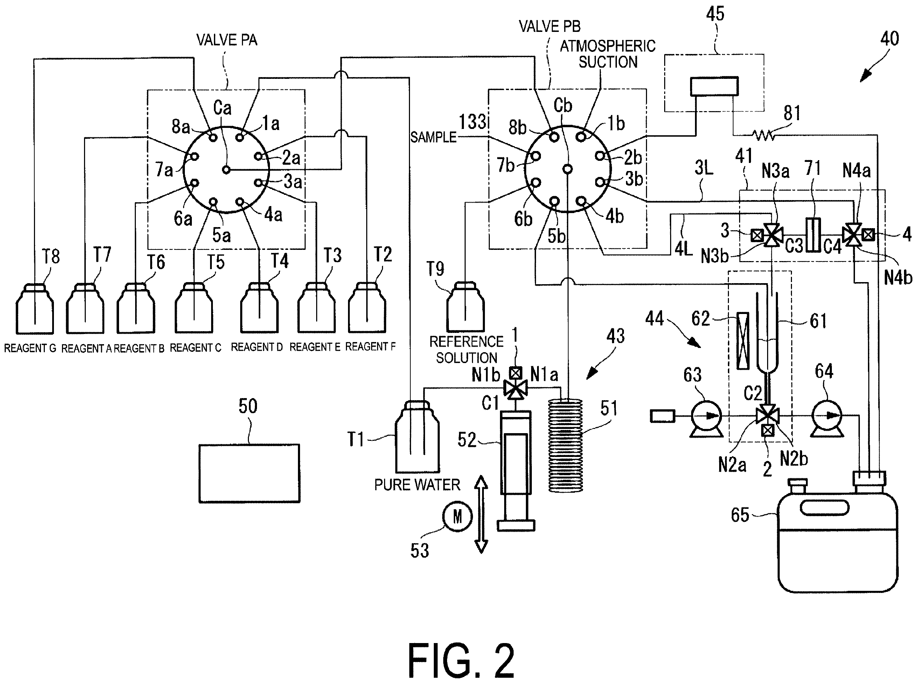

FIG. 2 is a diagram illustrating a schematic configuration of the analysis system 40. As illustrated in FIG. 2, the analysis system 40 includes, as a main configuration, a valve PA, a valve PB, a syringe portion 43, a collection unit 41, a reaction unit 44, a detection unit 45, and a control device 50.

With the valve PA, one of a plurality of reagents is selected, and a flow path is formed. In other words, with the valve PA, the flow path switching is performed. The reagent is a chemical used for analysis, such as a coloring reagent for detecting absorbance for example. In the present embodiment, an example of a case is described where a reagent A to a reagent F are used.

The reagent A is an aqueous ascorbic acid solution. The reagent B is nitric acid. The reagent C is an aqueous phenanthroline solution. The reagent D is an aqueous ammonium acetate solution. The reagent E is methanol. The reagent F is an aqueous ammonium acetate solution. The number and types of reagents used are not limited to the above.

In the valve PA, ports 1a to 8a serve as selection ports, and any one of the ports 1a to 8a selectively opens to a common port Ca. In other words, any one of the ports 1a to 8a selected is connected to the common port Ca. The port 1a is connected to a tank T1 storing pure water. The port 2a is connected to a tank T2 storing the reagent F. The port 3a is connected to a tank T3 storing the reagent E. The port 4a is connected to a tank T4 storing the reagent D. The port 5a is connected to a tank T5 storing the reagent C. The port 6a is connected to a tank T6 storing the reagent B. The port 7a is connected to a tank T7 storing the reagent A. The port 8a is connected to a tank T8 storing a reagent G. The reagent G is an aqueous sodium hydroxide solution. The reagent G can be used, for example, for the regeneration of iron adsorption cartridges.

The common port Ca of the valve PA is connected to the valve PB (port 8b). Thus, with the valve PA, any one of the reagents is selected to be supplied to the valve PB.

Thus, all of the reagents are selectable on the valve PA side. Thus, one reagent can be more reliably selected to be used for the analysis.

With the valve PB, one of a plurality of paths (ports 1b to 8b) is selected, and a flow path is formed. Thus, with the valve PB, flow path switching is performed, as with the valve PA.

In the valve PB, the ports 1b to 8b serve as selection ports, and any one of the ports 1b to 8b selectively opens to a common port Cb. In other words, any one of the ports 1b to 8b selected is connected to the common port Cb. The port 1b is open to allow atmospheric suction. The port 2b is connected to the detection unit 45. The port 3b is connected to the collection unit 41 through a line 3L. The port 4b is connected to the collection unit 41 through a line 4L. The port 5b is connected to the reaction unit 44. The port 6b is connected to a reference solution tank T9 storing a reference solution. The port 7b is connected to the provision line 133 to which the sample is supplied. The port 8b is connected to the common port Ca of the valve PA.

The common port Ca of the valve PA is connected to the syringe portion 43. Thus, with the valve PA, any one of the paths (ports 1b to 8b) is selected to open to the syringe portion 43.

The syringe portion 43 performs suction and delivery of liquid. For this purpose, the syringe portion 43 includes a sample loop 51 and a syringe pump 52.

The sample loop 51 is a vessel that temporarily stores liquid (or gas). Specifically, the sample loop 51 has one end connected to the common port Cb of the valve PB, and has the other end connected to the syringe pump 52 via a three-way valve 1. The sample loop 51 is configured by a tube in a coil shape in which liquid entered from the one end side can be temporarily stored. The one end side is connected to the common port Cb of the valve PB. Thus, various chemical solutions and samples in the valve PA, as well as air, and the like can be sucked in and stored.

The sample loop 51 has a coil shape in the present embodiment, but is not limited to the coil shape, as long as a vessel for which suction and sending can be performed with the syringe pump 52 is formed.

The syringe pump 52 sucks a predetermined amount of liquid (or gas) into the sample loop 51. The syringe pump 52 sends a predetermined amount of liquid (gas) from the sample loop 51. Thus, the syringe pump 52 implements suction into the sample loop 51 and sending from the sample loop 51. The syringe pump 52 changes a pressure state inside the sample loop 51, to perform the suction and the sending.

Specifically, the suction and the sending can be implemented by pushing and pulling an internal cylinder in a state where an external cylinder of the syringe pump 52 is fixed. The internal cylinder is driven by a motor 53. The liquid and the like can be sucked and sent highly accurately, based on the amount of pushing/pulling operation.

The syringe pump 52 has an outlet connected to C1 of the three-way valve 1. The three-way valve 1 has terminals C1, N1a, and N1b, with N1a connected to one end of the sample loop 51, and N1b connected to the tank T1. Any one of N1a and N1b of the three-way valve 1 is connected to C1.

The collection unit 41 includes a three-way valve 3, a three-way valve 4, and a cartridge 71. The collection unit 41 collects soluble iron (Fe) contained in the sample, and discharges other components.

The three-way valve 3 includes terminals C3, N3a, and N3b, with C3 connected to one end side of the cartridge 71, N3a connected to the port 4b of the valve PB through the line 4L, and N3b connected to a reaction vessel 61 of the reaction unit 44. Any one of N3a and N3b of the three-way valve 3 is connected to C3.

The three-way valve 4 includes terminals C4, N4a, and N4b, with C4 connected to the other end side of the cartridge 71, N4a connected to the port 3b of the valve PB through the line 3L, and N4b connected to a waste liquid tank 65. Any one of N4a and N4b of the three-way valve 4 is connected to C4.

The cartridge 71 is a chelating resin cartridge. Specifically, the cartridge 71 is formed by a solid phase extractant using a chelating resin. When the sample passes through the cartridge 71, the soluble iron is adsorbed, separated, and collected. The collected soluble iron is supplied to the reaction unit 44.

The reaction unit 44 produces a reaction solution. Specifically, the reaction unit 44 includes the reaction vessel 61, and the reaction solution is produced when a sample (soluble iron) and a chemical solution are supplied and mixed in the reaction vessel 61. The reaction vessel 61 is provided with a heater 62 that controls the temperature in the reaction vessel 61 to be suitable for facilitating the reaction.

A three-way valve 2 is connected to a lower portion of the reaction vessel 61. The three-way valve 2 includes terminals C2, N2a, and N2b, with C2 connected to the lower portion of the reaction vessel 61, N2a connected to a pump 63, and N2b connected to a waste liquid pump 64. Any one of N2a and N2b of the three-way valve 2 is connected to C2.

The pump 63 supplies air to the reaction vessel 61. When the air is supplied to the reaction vessel 61, air bubbling occurs, whereby the soluble iron and the reagent in the vessel are stirred and mixed. The waste liquid pump 64 discharges unwanted substances in the reaction vessel 61, to the waste liquid tank 65.

The detection unit 45 is supplied with a reaction solution, and detects the absorbance (optical density). The absorbance is an index indicating how much the intensity of light decreases when the light passes through a substance. As described below, the iron concentration can be estimated based on the absorbance. The detected absorbance is output to the control device 50 described below.

The reaction solution discharged from the detection unit 45 is discharged to the waste liquid tank 65 through a resistance tube 81.

The control device 50 controls various devices in the analysis system 40. Specifically, various valves and pumps, the syringe pump 52 (motor 53), three-way valves, the heater 62, and the like are controlled to perform the analysis. Preferably, the control device 50 performs the control to perform the analysis automatically. It is preferable that automatic calibration or automatic washing is performed.

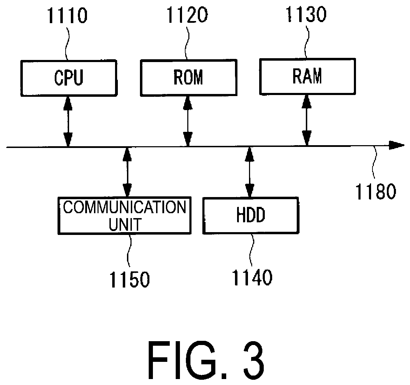

FIG. 3 is a view illustrating an example of the hardware configuration of the control device 50 according to the present embodiment.

As illustrated in FIG. 3, the control device 50 is a computer system, and includes, for example, a CPU 1110, a Read Only Memory (ROM) 1120 for storing programs or the like to be executed by the CPU 1110, a Random Access Memory (RAM) 1130 functioning as a work area when each program is executed, a hard disk drive (HDD) 1140 as a mass storage device, and a communication unit 1150 for connecting to a network or the like. Note that a solid state drive (SSD) may be used as the mass storage device. These portions are interconnected via a bus 1180.

The control device 50 may include an input unit including a keyboard, a mouse, and the like and a display unit including a liquid crystal display device and the like for displaying data.

The storage medium for storing the program or the like executed by the CPU 1110 is not limited to the ROM 1120. For example, another auxiliary storage device such as a magnetic disk, a magneto-optical disk, or a semiconductor memory may be used.

A series of processing steps for achieving various functions to be described later is recorded in the hard disk drive 1140 or the like in the form of a program, and the CPU 1110 reads the program and writes it to the RAM 1130 or the like to execute processing and arithmetic processing of information. This allows various functions to be described below to be achieved. As the program, a program pre-installed in the ROM 1120 or another storage medium, a program provided in a state of being stored in a computer-readable storage medium, a program distributed through wired or wireless communication methods, or the like may also be used. Examples of the computer-readable storage medium include a magnetic disk, a magneto-optical disk, a CD-ROM, a DVD-ROM, and a semiconductor memory.

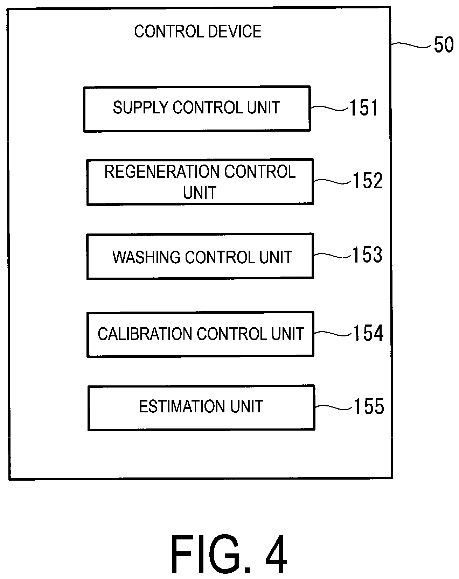

FIG. 4 is a functional block diagram illustrating functions of the control device 50. As illustrated in FIG. 4, the control device 50 includes a supply control unit 151, a regeneration control unit 152, a washing control unit 153, a calibration control unit 154, and an estimation unit 155.

The supply control unit 151 controls the valve PA, the valve PB, the three-way valves 1 to 4, the syringe pump 52, and the like, to control transportation of reagents and samples. For example, when the sample is supplied to the collection unit 41, the port 7b of the valve PB is selected to suck the sample into the sample loop 51 through via the valve PB, and the port 4b of the valve PB is selected to send the sample to the collection unit 41 via the valve PB. As described above, the flow path switching with the valve PA and the valve PB is controlled in such a manner that the chemical solution and the like are temporarily stored in the sample loop 51, and then are sent to the collection unit 41. Thus, the supply control unit 151 forms a sample supply tube through which the sample is supplied to the collection unit 41, and the sample is supplied to the collection unit 41 through the sample supply tube.

With the port 5b of the valve PB selected, the reaction solution in the reaction vessel 61 can be sucked into the sample loop 51 via the valve PB. With the port 2b of the valve PB then selected, the reaction solution in the sample loop 51 can be sent to the detection unit 45.

Thus, the liquid is transported through the sample loop 51. The sample loop 51 is controlled with the syringe pump 52, and thus the amount transported can be adjusted highly accurately.

The supply control unit 151 further controls the collection unit 41 to extract the soluble iron in the sample, and then send the soluble iron to the reaction unit 44. Specifically, the supply control unit 151 supplies the soluble iron collected by the collection unit 41 and the reagent to the reaction unit 44, to produce the reaction solution. The supply control unit 151 supplies the sample to the collection unit 41 through the sample supply tube, then forms an eluate supply tube through which an eluate is supplied to the collection unit 41, sends the eluate to the collection unit 41 through the eluate supply tube, and supplies the soluble iron to the reaction unit 44 together with the eluate. The eluate is a solution for sending the collected soluble iron to the reaction unit 44. The detailed flow will be described below.

The supply control unit 151 makes the pump 63 supply air to the reaction unit 44, causing the air bubbling to stir the soluble iron and the reagent. Specifically, after the soluble iron and the reagent have been supplied into the reaction vessel 61, the pump 63 sends the air in a state where C2 and N2a are connected in the three-way valve 2. Through the air bubbling, the stirring can be effectively performed. For example, the devices can be expected to have longer service life than that achieved by a propeller system.

The regeneration control unit 152 supplies an alkaline solution to the collection unit 41, after supplying the soluble iron to the reaction unit 44. The retention capacity can be regenerated by supplying the alkaline solution to the chelating resin. Thus, the regeneration processing is executed for the collection unit 41, after the collecting of the soluble iron and supplying the soluble iron to the reaction unit 44 have been completed.

The alkaline solution is, for example, an aqueous sodium hydroxide solution. Thus, the regeneration control unit 152 forms an alkaline solution supply tube through which the alkaline solution is supplied to the collection unit 41.

The washing control unit 153 supplies pure water to the reaction unit 44 and/or the detection unit 45. The pure water is stored in the tank T1. By controlling the valve PB, washing can be performed with the pure water supplied from the tank T1 to the reaction unit 44 and the detection unit 45. The pure water control unit is not limited to the configuration of supplying the pure water in the tank T1 to the reaction unit 44 and the detection unit 45. The washing may be performed with the pure water flowing in other devices and tubes. Thus, the washing control unit 153 forms a pure water supply pipe through which the pure water is supplied from a pure water tank to the reaction unit 44, the detection unit 45, and the like, and supplies the pure water through the pure water supply pipe.

The calibration control unit 154 calibrates the detection unit 45, based on a reaction solution (reference reaction solution) obtained by mixing the reference solution and the reagent. The reference solution is a solution in which the iron concentration is adjusted in advance. Thus, the iron concentration of the reference solution is known. Thus, through processing similar to the analysis on the sample executed using the reference solution instead of the sample, the reaction solution using the reference solution can be prepared. Then, the reaction solution is sent to the detection unit 45 and the absorbance is detected. Thus, the iron concentration of the reference solution the iron concentration of which is known can be analyzed. When the analysis result for the known iron concentration is outside a predetermined reference range, the detection unit 45 (or the estimation unit 155) is calibrated, so that degradation of the analysis accuracy can be prevented.

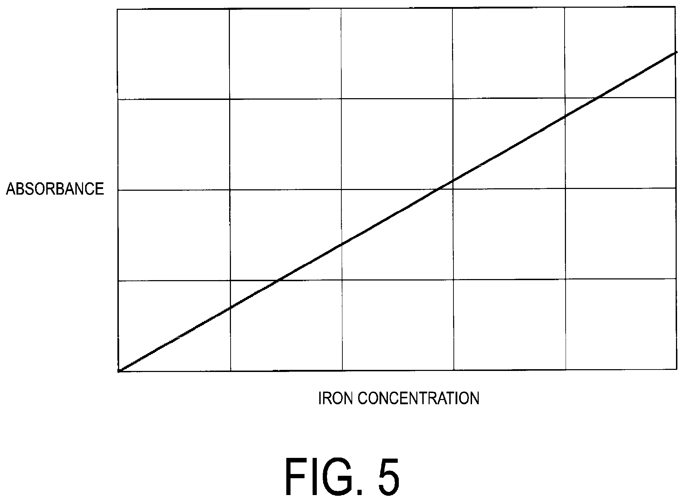

The estimation unit 155 estimates the iron concentration in the sample based on the detected absorbance. Thus, the estimation unit 155 acquires the detection result of the absorbance from the detection unit 45. The absorbance is correlated with the iron concentration. FIG. 5 is a diagram illustrating an example of the relationship between absorbance and iron concentration. The relationship as illustrated in FIG. 5 can be established through pre-testing on a sample including soluble iron and the like.

The estimation unit 155 estimates the iron concentration based on the absorbance detected, by using the relationship between the absorbance and the iron concentration as illustrated in FIG. 5. The relationship between the absorbance and the iron concentration illustrated in FIG. 5 is an example, and should not construed in a limiting sense.

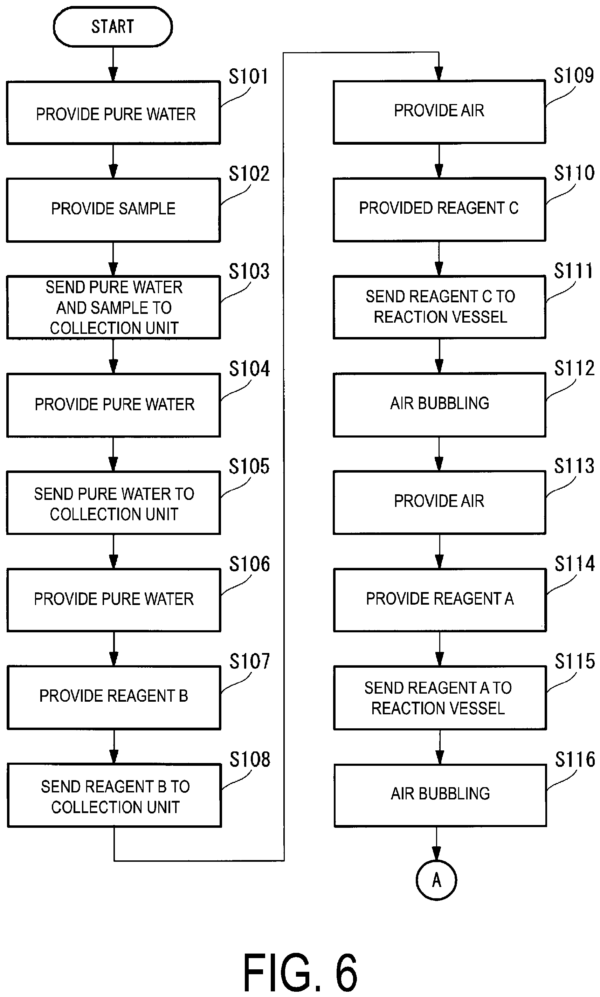

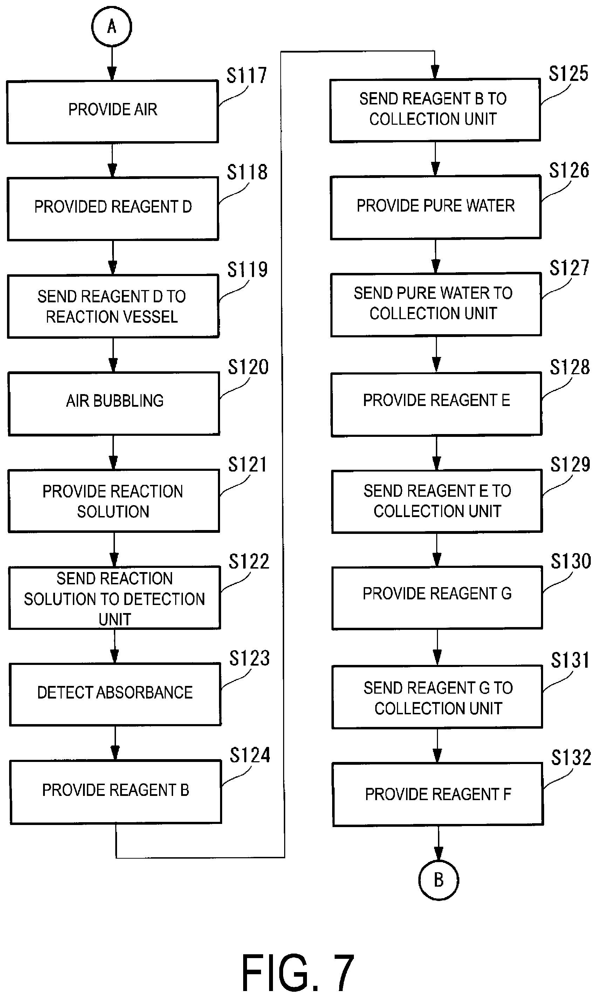

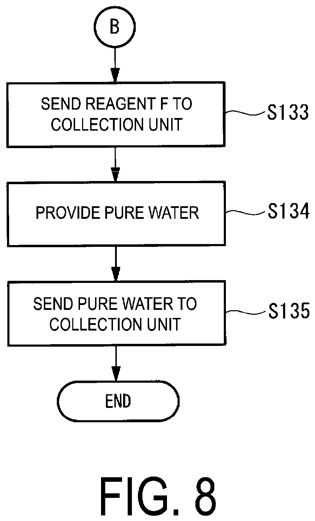

Next, an example of the analysis processing by the control device 50 described above will be described with reference to FIG. 6, FIG. 7, and FIG. 8. FIG. 6, FIG. 7, and FIG. 8 are flowcharts illustrating an example of a procedure of the analysis processing according to the present embodiment. In the flow illustrated in FIG. 6, FIG. 7, and FIG. 8. the processing is continuously executed between portions A and B, meaning that S117 in FIG. 7 is executed after S116 in FIG. 6 is executed, and S133 in FIG. 8 is executed after S132 in FIG. 7 is executed. The flow illustrated in FIG. 6, FIG. 7, and FIG. 8 is executed at a predetermined timing when the analysis is executed for example. The analysis may be executed with the flow illustrated in FIG. 6, FIG. 7, and FIG. 8 executed at an interval set in advance. The flow illustrated in FIG. 6, FIG. 7, and FIG. 8 is an example, the processing is not limited to the flow illustrated in FIG. 6, FIG. 7, and FIG. 8. as long as the absorbance is detected for the reaction solution produced by mixing the sample and the reagent.

First of all, the port 1a of the valve PA is selected and the port 8b of the valve PB is selected, to provide a predetermined amount of pure water into the sample loop 51 (S101).

Then, the port 7b of the valve PB is selected to provide a predetermined amount of sample into the sample loop 51 (S102).

Then, in a state where the port 4b of the valve PB is selected, N3a of the three-way valve 3 is selected, and N4b of the three-way valve 4 is selected, the pure water and the sample are sent to the collection unit 41 (S103). The sample passes through the cartridge 71 as a result of S103, and thus the soluble iron is collected in the cartridge 71. The other components of the sample are discharged to the waste liquid tank 65.

Then, the port 1a of the valve PA is selected and the port 8b of the valve PB is selected, to provide a predetermined amount of pure water into the sample loop 51 (S104).

Then, in a state where the port 4b of the valve PB is selected, N3a of the three-way valve 3 is selected, and N4b of the three-way valve 4 is selected, the pure water is sent to the collection unit 41 (S105). As a result, the cartridge 71 is purged.

Then, the port 1a of the valve PA is selected and the port 8b of the valve PB is selected, to provide a predetermined amount of pure water into the sample loop 51 (S106).

Then, the port 6a of the valve PA is selected and the port 8b of the valve PB is selected, to provide a predetermined amount of reagent B into the sample loop 51 (S107).

Then, in a state where the port 3b of the valve PB is selected, N3b of the three-way valve 3 is selected, and N4a of the three-way valve 4 is selected, the reagent B is sent to the collection unit 41 (S108). As a result, the reagent B passes through the cartridge 71 in a direction opposite to that in which the sample is supplied. Thus, the soluble iron collected by reagent B is separated, and the reagent B and the soluble iron are supplied to the reaction vessel 61. Thus, the reagent B serves as the eluate.

Then, the port 1b of the valve PB is selected to provide a predetermined amount of air to the sample loop 51 (S109).

Then, the port 5a of the valve PA is selected and the port 8b of the valve PB is selected, to provide a predetermined amount of reagent C into the sample loop 51 (S110).

Then, the port 5b of the valve PB is selected, and the reagent C is sent to the reaction vessel 61 (S111).

Stirring in the reaction vessel 61 is then performed by the air bubbling (S112).

Then, the port 1b of the valve PB is selected to provide a predetermined amount of air to the sample loop 51 (S113).

Then, the port 7a of the valve PA is selected and the port 8b of the valve PB is selected, to provide a predetermined amount of reagent A into the sample loop 51 (S114).

Then, the port 5b of the valve PB is selected, and the reagent A is sent to the reaction vessel 61 (S115).

Stirring in the reaction vessel 61 is then performed by the air bubbling (S116).

Then, the port 1b of the valve PB is selected to provide a predetermined amount of air to the sample loop 51 (S117).

Then, the port 4a of the valve PA is selected and the port 8b of the valve PB is selected, to provide a predetermined amount of reagent D into the sample loop 51 (S118).

Then, the port 5b of the valve PB is selected, and the reagent D is sent to the reaction vessel 61 (S119).

Stirring in the reaction vessel 61 is then performed by the air bubbling (S120).

Thus, the sample (soluble iron) and reagent are mixed in the reaction vessel 61, whereby the reaction solution is produced.

Then, the port 5b of the valve PB is selected to provide a predetermined amount of reaction solution into the sample loop 51 (S121).

Then, the port 2b of the valve PB is selected to send a predetermined amount of reaction solution from the sample loop 51 to the detection unit 45 (S122).

Then, the detection unit 45 detects the absorbance of the reaction solution (S123).

Then, the port 6a of the valve PA is selected and the port 8b of the valve PB is selected, to provide a predetermined amount of reagent B into the sample loop 51 (S124).

Then, in a state where the port 3b of the valve PB is selected, N3b of the three-way valve 3 is selected, and N4a of the three-way valve 4 is selected, the reagent B is sent to the collection unit 41 (S 125). As a result, the cartridge 71 is washed by the reagent B.

Then, the port 1a of the valve PA is selected and the port 8b of the valve PB is selected, to provide a predetermined amount of pure water into the sample loop 51 (S126).

Then, in a state where the port 3b of the valve PB is selected, N3b of the three-way valve 3 is selected, and N4a of the three-way valve 4 is selected, the pure water is sent to the collection unit 41 (S127). As a result, the cartridge 71 is washed by the pure water.

Then, the port 3a of the valve PA is selected and the port 8b of the valve PB is selected, to provide a predetermined amount of reagent E into the sample loop 51 (S128).

Then, in a state where the port 3b of the valve PB is selected, N3b of the three-way valve 3 is selected, and N4a of the three-way valve 4 is selected, the reagent E is sent to the collection unit 41 (S 129). As a result, the cartridge 71 is regenerated by the reagent E. Specifically, the washing is performed to remove, using the reagent E, organic substances remaining attached to the cartridge 71.

Then, the port 8a of the valve PA is selected and the port 8b of the valve PB is selected, to provide a predetermined amount of reagent G into the sample loop 51 (S130).

Then, in a state where the port 3b of the valve PB is selected, N3b of the three-way valve 3 is selected, and N4a of the three-way valve 4 is selected, the reagent G is sent to the collection unit 41 (S131). As a result, the cartridge 71 is regenerated by the reagent G.

Then, the port 2a of the valve PA is selected and the port 8b of the valve PB is selected, to provide a predetermined amount of reagent F into the sample loop 51 (S132).

Then, in a state where the port 3b of the valve PB is selected, N3b of the three-way valve 3 is selected, and N4a of the three-way valve 4 is selected, the reagent F is sent to the collection unit 41 (S133). As a result, the cartridge 71 is regenerated by the reagent F. Thus, pH adjustment is performed in the cartridge 71 using the reagent F.

Then, the port 1a of the valve PA is selected and the port 8b of the valve PB is selected, to provide a predetermined amount of pure water into the sample loop 51 (S134).

Then, in a state where the port 3b of the valve PB is selected, N3b of the three-way valve 3 is selected, and N4a of the three-way valve 4 is selected, the pure water is sent to the collection unit 41 (S 135). As a result, the cartridge 71 is washed by the pure water. The solution for washing and regeneration is sent to the reaction vessel 61, and is discharged to the waste liquid tank 65.

As described above, the absorbance of the sample is detected under the control by the control device 50. The detected absorbance is output to the control device 50, and the iron concentration is identified. The iron concentration may be identified using the detected absorbance, by manual analysis by an operator, or the like. The amount of the added iron chloride input is controlled with the iron concentration monitored in the wastewater treatment facility 120. Thus, the iron chloride can be prevented from being excessively input.

In the flow of the sample analysis in FIG. 6, FIG. 7, and FIG. 8, the analysis can be executed on the reference solution, by providing the reference solution instead of the sample. In this case, in S102, the port 6b of the valve PB may be selected to provide a predetermined amount of reference solution to the sample loop 51. The iron concentration of the reference solution is analyzed with the other processing executed as in FIG. 6, FIG. 7, and FIG. 8. The calibration control unit 154 executes the calibration processing if a comparison between the iron concentration of the reference solution thus analyzed and the known iron concentration (reference solution iron concentration) of the reference solution indicates the analyzed iron concentration is different from the reference iron concentration by a predetermined value or more. Thus, the degradation of the analysis accuracy can be prevented.

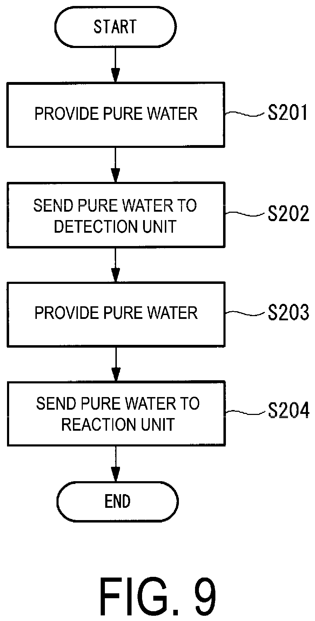

Next, an example of the washing processing by the control device 50 described above will be described with reference to FIG. 9. FIG. 9 is a flowchart illustrating an example of a procedure of the washing processing according to the present embodiment. The flow illustrated in FIG. 9 is executed, for example, before or after the start of the analysis.

First of all, the port 1a of the valve PA is selected and the port 8b of the valve PB is selected, to provide a predetermined amount of pure water into the sample loop 51 (S201).

Then, the port 2b of the valve PB is selected to send the pure water from the sample loop 51 to the detection unit 45 (S202). Thus, the detection unit 45 is washed. The pure water that has passed through the detection unit 45 is discharged to the waste liquid tank 65.

Then, the port 1a of the valve PA is selected and the port 8b of the valve PB is selected, to provide a predetermined amount of pure water into the sample loop 51 (S203).

Then, the port 5b of the valve PB is selected to send the pure water from the sample loop 51 to the reaction unit 44 (reaction vessel 61) (S204). Thus, the reaction unit 44 is washed. The pure water that has passed through the reaction unit 44 is discharged to the waste liquid tank 65.

In this manner, the detection unit 45 and the reaction unit 44 are washed. The order of washing of the detection unit 45 and the reaction unit 44 is not limited. Furthermore, only one of the detection unit 45 and the reaction unit 44 may be washed. Preferably, the reaction unit 44 is washed for a plurality of times (three times for example). When the washing is performed for a plurality of times, the pure water is preferably discharged each time the washing is performed.

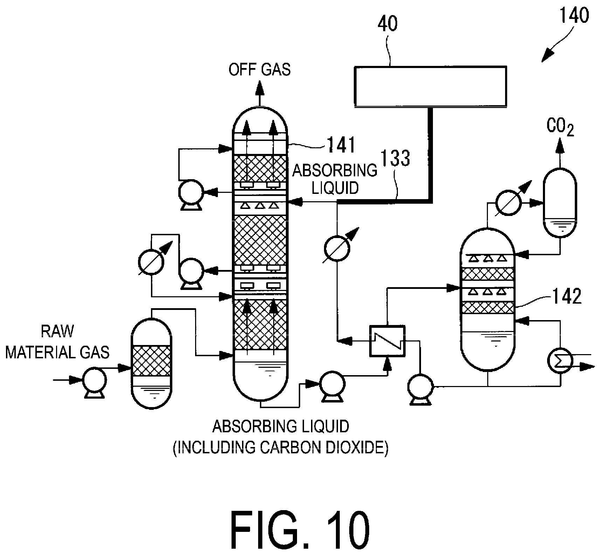

While a case where the analysis system 40 is applied to the wastewater treatment facility 120 is described in the present embodiment, the applicable facility is not limited to the wastewater treatment facility 120. For example, the application target may be a carbon dioxide recovery facility 140 illustrated in FIG. 10. Specifically, in the carbon dioxide recovery facility 140, raw material gas including carbon dioxide and absorbing liquid (for example, an aqueous alkaline solution such as amine) are brought into contact with each other in an absorption tower 141. Thus, carbon dioxide is selectively absorbed in the absorbing liquid. The raw material gas is discharged as off gas after coming into contact with the absorbing liquid. The absorbing liquid that has absorbed the carbon dioxide is subjected to heating processing in a regeneration tower 142, whereby carbon dioxide is separated. The carbon dioxide thus separated is recovered. The absorbing liquid from which carbon dioxide is separated is again supplied to the absorption tower 141.

In such a carbon dioxide recovery facility 140, the iron concentration of the absorbing liquid is preferably monitored, for quality management for the absorbing liquid, checking of the corrosion status, and the like. Thus, part of the absorbing liquid supplied to the absorption tower 141 may be provided into the analysis system 40 as a sample to be analyzed. By analyzing the iron concentration of the absorbing liquid, for example, it is possible to check whether rust exists, rust is increasing, or the like.

As described above, with the analysis system and the management system, the analysis method, and the analysis program according to the present embodiment, the absorbance can be detected for the reaction solution produced using a chemical solution and soluble iron included in a sample. Thus, the absorbance can be accurately detected with respect to the soluble iron of the sample. For example, when a component different from the soluble iron is contained in the sample, the component may inhibit the reaction between the soluble iron and the reagent to affect the absorbance detection. Still, with the soluble iron collected, the impact of the component can be suppressed.

With the sample supply tube and the eluate supply tube provided, the soluble iron and the eluate are supplied to the reaction unit 44 with the eluate making the soluble iron that has been collected by the collection unit 41 flow. Thus, the collected soluble iron can be more reliably supplied to the reaction unit 44.

The retention capacity of the chelating resin can be regenerated by supplying the alkaline solution to the collection unit 41.

The syringe pump 52 sends the sample to the collection unit 41, so that the amount of the sample sent can be controlled with high accuracy. Thus, reduction in the used amount of the sample can be expected.

The soluble iron and the reagent are stirred by air bubbling with the air supplied into the reaction unit 44 by the pump 63, and thus can be more reliably mixed.

The washing can be performed with the pure water supplied to the reaction unit 44 and/or the detection unit 45.

The detection unit 45 can execute the calibration based on the reaction solution obtained by mixing the reference solution the iron concentration of which has been adjusted in advance and the reagent, and thus with the iron concentration known.

### Second Embodiment

Next, an analysis system and a management system, an analysis method, and an analysis program according to a second embodiment of the present disclosure will be described below.

In the present embodiment, a case where the liquidity of the sample is automatically adjusted is described. An analysis system and a management system, an analysis method, and an analysis program according to the present embodiment will be described below while focusing on differences from the first embodiment.

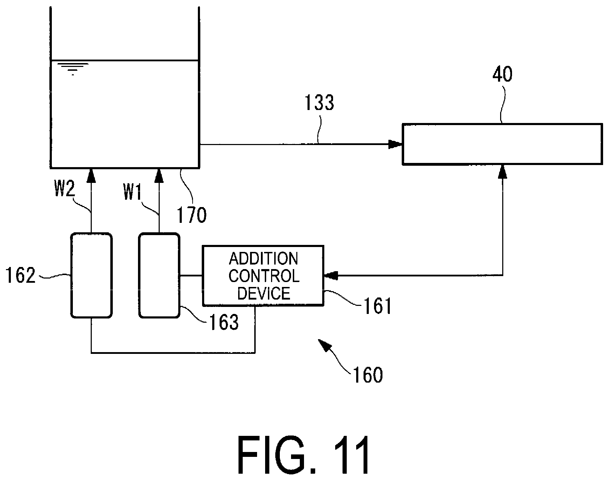

As illustrated in FIG. 11, the management system according to the present embodiment includes the analysis system 40 and an adjustment system 160. The analysis system 40 according to the present embodiment is assumed to be provided with a sample from a target facility 170. The target facility 170 is a facility from which the sample is provided.

The adjustment system 160 acquires the analysis result obtained by the analysis system 40 and adjusts the iron concentration of the sample in accordance with the analysis result. As illustrated in FIG. 11, the adjustment system 160 includes a tank 163, a tank 162, and an addition control device 161. In the adjustment system 160, the iron concentration or the absorbance correlated with the iron concentration may be used as the analysis result.

The tank 163 stores an iron concentration adjustment chemical solution. The tank 163 is connected to the target facility 170 through a supply line W1. Thus, the iron concentration adjustment chemical solution is added to a solution in the target facility 170. The supply line W1 is provided with an electromagnetic valve (not illustrated), a flow meter (not illustrated), and a pump (not illustrated). The electromagnetic valve and the pump are controlled by the addition control device 161 described below. A result of measurement by the flow meter is transmitted to the addition control device 161.

The tank 162 stores pure water. The tank 162 is connected to the target facility 170 through a supply line W2. Thus, the pure water is added to a solution in the target facility 170. The supply line W2 is provided with an electromagnetic valve (not illustrated), a flow meter (not illustrated), and a pump (not illustrated). The electromagnetic valve and the pump are controlled by the addition control device 161 described below. A result of measurement by the flow meter is transmitted to the addition control device 161.

The addition control device 161 controls the amount of the addition in accordance with the analysis result obtained by the analysis system 40. For example, the addition control device 161 is formed of computer system (computing system) as illustrated in FIG. 3, as in the case of the control device 50. The control device 50 and the addition control device 161 may be formed of different computer systems, or may be integrated into a single computer system.

The addition control device 161 adjusts the iron concentration by adjusting the amount of the iron concentration adjustment chemical solution input. With the iron concentration adjustment chemical solution input, the iron concentration of the solution is controlled to be within the predetermined reference range. For example, the iron concentration adjustment chemical solution is input by the pump while the input amount is observed using the flow meter with the electromagnetic valve open. The method for adjusting iron concentration is not limited to the above. For example, the input amount may be determined according to the detected value of the iron concentration. The iron concentration of the solution may be adjusted by inputting the pure water.

The management system may be provided with a notification system (not illustrated). The notification system acquires the analysis result from the analysis system 40, and issues a notification indicating abnormality when the analysis result is outside a management reference range set in advance. The notification may be issued in a facility provided with the target facility 170, or may be issued through transmission of information to a remote facility. With the notification indicating abnormality issued when the result is outside the management reference range, it is possible to prevent the abnormal state from continuing without being addressed.

As described above, with the analysis system and the management system, the analysis method, and the analysis program according to the present embodiment, the iron concentration is adjusted in accordance with the analysis result, so that the liquidity of the sample can be automatically adjusted. Thus, a human error caused by an operator or the like can be prevented, and work time can be shortened.

### Reference Signs List

1 to 4 Three-way valve
1a to 8a Port
1b to 8b Port
40 Analysis system
41 Collection unit
43 Syringe portion
44 Reaction unit
45 Detection unit
50 Control device
51 Sample loop
52 Syringe pump
53 Motor
61 Reaction vessel
62 Heater
63 Pump
64 Waste liquid pump
65 Waste liquid tank
71 Cartridge
81 Resistance tube
120 Wastewater treatment facility
121 Discharge water tank
122 First neutralization tank
123 Second neutralization tank
124 Floc tank
125 Floc sedimentation tank
127 Sludge tank
128 Acid Storage
129 Caustic soda storage
130 Flocculant dissolution storage
131 Filter apparatus
132 Discharge line
133 Provision line
140 Carbon dioxide recovery facility
141 Absorption tower
142 Regeneration tower
151 Supply control unit
152 Regeneration control unit
153 Washing control unit
154 Calibration control unit
155 Estimation unit
160 Adjustment system
161 Addition control device
162 Tank
163 Tank
170 Target facility
1110 CPU
1120 ROM
1130 RAM
1140 Hard disk drive
1150 Communication unit
1180 Bus
A to G Reagent
Ca and Cb Common Port
PA to PB Valve
T1 to T9 Tank
W1 and W2 Supply line

## Claims

1. A management system for a wastewater treatment facility (120, 170) or a carbon dioxide recovery facility (140, 170), the system comprising
an analysis system (40) applied to the discharge line (132) of the treatment facility (120); and
an adjustment system (160), wherein the analysis system (40) comprises:
a collection unit (41) configured to collect soluble iron contained in a sample;
a reaction unit (44) configured to produce a reaction solution;
a detection unit (45) configured to detect an absorbance of the reaction solution;
a supply control unit (151) configured to supply the soluble iron collected in the collection unit (41) and a reagent to the reaction unit (44),
an estimation unit (155) configured to estimate the iron concentration in the sample based on the detected absorbance and;
wherein the adjustment system (160) is configured to acquire an analysis result obtained by the analysis system (40) and is configured to adjust the iron concentration of the sample in accordance with the analysis result; the adjustment system (160) includes a tank (163) that is configured to store an iron concentration adjustment chemical solution and is connected to the target facility (170) through a supply line (W1) so that the iron concentration adjustment chemical solution is added to a solution in the target facility (170); the adjustment system (160) includes an addition control device (161) that is configured to control the amount of the addition in accordance with the analysis result obtained by the analysis system (40).

2. The management system according to claim 1, wherein the collection unit (41) is formed by a solid phase extractant using a chelating resin.

3. The management system according to claim 1 or 2 further comprising:
a sample supply tube through which the sample is supplied to the collection unit (41); and
an eluate supply tube through which an eluate is supplied to the collection unit (41), wherein
the supply control unit (151) is configured to supplly the sample to the collection unit (41) through the sample supply tube, then is configured to send the eluate to the collection unit (41) through the eluate supply tube, and is configured to supplly the eluate and the soluble iron to the reaction unit (44).

4. The management system according to claim 2 further comprising:
an alkaline solution supply tube through which an alkaline solution is supplied to the collection unit (41); and
a regeneration control unit (152) configured to supply the soluble iron to the reaction unit (44), and then supply the alkaline solution to the collection unit (41).

5. The management system according to any one of claims 1 to 4 further comprising a syringe pump (52) configured to suck liquid into a vessel and send a predetermined amount of the liquid from the vessel, wherein
the supply control unit (151) is configured to control the syringe pump (52) to send the sample to the collection unit (41).

6. The management system according to any one of claims 1 to 5 further comprising a pump (63) configured to supply air to the reaction unit (44), wherein
the supply control unit (151) is configured to make the pump (63) supply the air to the reaction unit (44), causing air bubbling to stir the soluble iron and the reagent.

7. The management system according to any one of claims 1 to 6 further comprising:
a pure water tank (T1) storing pure water;
a pure water supply pipe through which the pure water is supplied from the pure water tank (T1) to one of the reaction unit (44) and the detection unit (45) or both; and
a washing control unit (153) configured to supply the pure water to one of the reaction unit (44) and the detection unit (45) or both through the pure water supply pipe.

8. The management system according to any one of claims 1 to 7 further comprising:
a reference solution tank (T9) storing a reference solution iron concentration of which is adjusted in advance; and
a calibration control unit (154) configured to calibrate the detection unit (45) based on the reaction solution obtained by mixing the reference solution and the reagent.

9. The management system according to any one of claims 1 to 8 further comprising a notification system configured to issue a notification indicating abnormality, when the analysis result is outside a management reference range set in advance.

10. A managing method for a wastewater treatment facility (120, 170) or a carbon dioxide recovery facility (140, 170) performing in an analysis system (40) applied to the discharge line (132) of the treatment facility (120) comprising the following steps:
- collecting soluble iron contained in a sample;
- supplying a reagent to the sample to produce a reaction solution;
- detecting an absorbance of the reaction solution;
estimating the iron concentration in the sample based on the detected absorbance thus providing an analysis result;
adjusting the iron concentration of the sample in accordance with the analysis result by:
storing an iron concentration adjustment chemical solution and
adding the iron concentration adjustment chemical solution to a solution in the target facility (170);
controlling the amount of the addition in accordance with the analysis result obtained by the analysis system (40).

## Patentansprüche

1. Managementsystem für eine Abwasserbehandlungsanlage (120, 170) oder eine Kohlendioxidrückgewinnungsanlage (140, 170), wobei das System umfasst ein Analysesystem (40), das an die Auslassleitung (132) der Behandlungsanlage (120) angelegt bzw. angeschlossen ist; und
ein Einstellsystem (160),
wobei das Analysesystem (40) umfasst:
eine Sammeleinheit (41), die konfiguriert ist, in einer Probe enthaltenes lösliches Eisen zu sammeln;
eine Reaktionseinheit (44), die konfiguriert ist, eine Reaktionslösung herzustellen;
eine Detektionseinheit (45), die konfiguriert ist, einen Absorptionsgrad der Reaktionslösung zu detektieren;
eine Zufuhrsteuerungs- bzw. -regelungseinheit (151), die konfiguriert ist, das in der Sammeleinheit (41) gesammelte lösliche Eisen und ein Reagenz der Reaktionseinheit (44) zuzuführen;
eine Schätzeinheit (155), die konfiguriert ist, die Eisenkonzentration in der Probe basierend auf dem detektierten Absorptionsgrad zu schätzen; und
wobei das Einstellsystem (160) konfiguriert ist, ein durch das Analysesystem (40) gewonnenes Analyseergebnis zu erhalten, und konfiguriert ist, die Eisenkonzentration der Probe gemäß dem Analyseergebnis einzustellen; das Einstellsystem (160) einen Tank (163) enthält, der konfiguriert ist, eine chemische Lösung zur Eisenkonzentrationseinstellung zu speichern, und durch eine Zufuhrleitung (W1) mit der Zielanlage (170) verbunden ist, sodass die chemische Lösung zur Eisenkonzentrationseinstellung einer Lösung in der Zielanlage (170) hinzugegeben wird;
das Einstellsystem (160) eine Zugabesteuerungs- bzw. -regelungsvorrichtung (161) enthält, die konfiguriert ist, die Zugabemenge gemäß dem durch das Analysesystem (40) gewonnenen Analyseergebnis zu steuern bzw. zu regeln.

2. Managementsystem nach Anspruch 1, wobei die Sammeleinheit (41) durch ein Festphasenextraktionsmittel unter Verwendung eines Chelatbildnerharzes gebildet ist.

3. Managementsystem nach Anspruch 1 oder 2, ferner umfassend:
ein Probenzufuhrrohr, durch das die Probe der Sammeleinheit (41) zugeführt wird; und
ein Eluatzufuhrrohr, durch das ein Eluat der Sammeleinheit (41) zugeführt wird,
wobei
die Zufuhrsteuerungs- bzw. -regelungseinheit (151) konfiguriert ist, die Probe durch das Probenzufuhrrohr der Sammeleinheit (41) zuzuführen, dann konfiguriert ist, das Eluat durch das Eluatzufuhrrohr an die Sammeleinheit (41) zu schicken, und konfiguriert ist, das Eluat und das lösliche Eisen der Reaktionseinheit (44) zuzuführen.

4. Managementsystem nach Anspruch 2, ferner umfassend:
ein Zufuhrrohr für eine alkalische Lösung, durch das eine alkalische Lösung der Sammeleinheit (41) zugeführt wird; und
eine Regenerationssteuerungs- bzw. -regelungseinheit (152), die konfiguriert ist, das lösliche Eisen der Reaktionseinheit (44) zuzuführen und dann die alkalische Lösung der Sammeleinheit (41) zuzuführen.

5. Managementsystem nach einem der Ansprüche 1 bis 4, ferner umfassend eine Spritzenpumpe (52), die konfiguriert ist, Flüssigkeit in ein Gefäß zu saugen und eine vorbestimmte Menge der Flüssigkeit aus dem Gefäß zu schicken, wobei die Zufuhrsteuerungs- bzw. -regelungseinheit (151) konfiguriert ist, die Spritzenpumpe (52) zu steuern bzw. zu regeln, um die Probe an die Sammeleinheit (41) zu schicken.

6. Managementsystem nach einem der Ansprüche 1 bis 5, ferner umfassend eine Pumpe (63), die konfiguriert ist, der Reaktionseinheit (44) Luft zuzuführen, wobei
die Zufuhrsteuerungs- bzw. -regelungseinheit (151) konfiguriert ist, die Pumpe (63) zu veranlassen, der Reaktionseinheit (44) die Luft zuzuführen, wodurch Luftblasen bewirkt werden, die das lösliche Eisen und das Reagenz verrühren bzw. vermischen.

7. Managementsystem nach einem der Ansprüche 1 bis 6, ferner umfassend:
einen Reinwassertank (T1), der Reinwasser speichert;
eine Reinwasserzufuhrrohrleitung, durch die das Reinwasser von dem Reinwassertank (T1) einer der Reaktionseinheit (44) und der Detektionseinheit (45) oder beiden zugeführt wird; und
eine Waschsteuerungs- bzw. -regelungseinheit (153), die konfiguriert ist, das Reinwasser durch die Reinwasserzufuhrrohrleitung einer der Reaktionseinheit (44) und der Detektionseinheit (45) oder beiden zuzuführen.

8. Managementsystem nach einem der Ansprüche 1 bis 7, ferner umfassend:
einen Referenzlösungstank (T9), der eine Referenzlösung speichert, deren Eisenkonzentration im Voraus eingestellt wird; und
eine Kalibrierungssteuerungs- bzw. -regelungseinheit (154), die konfiguriert ist,
die Detektionseinheit (45) basierend auf der durch Mischen der Referenzlösung und des Reagenz gewonnenen Reaktionslösung zu kalibrieren.

9. Managementsystem nach einem der Ansprüche 1 bis 8, ferner umfassend ein Benachrichtigungssystem, das konfiguriert ist, eine Benachrichtigung auszugeben, die eine Anomalität angibt, wenn das Analyseergebnis außerhalb eines vorab festgelegten Managementreferenzbereichs liegt.

10. Managingverfahren für eine Abwasserbehandlungsanlage (120, 170) oder eine Kohlendioxidrückgewinnungsanlage (140, 170), das in einem Analysesystem (40) arbeitet, das an die Auslassleitung (132) der Behandlungsanlage (120) angelegt bzw. angeschlossen ist, umfassend die folgenden Schritte:
- Sammeln von in einer Probe enthaltenem löslichen Eisen;
- Zuführen eines Reagenz zu der Probe zum Herstellen einer Reaktionslösung;
- Detektieren eines Absorptionsgrads der Reaktionslösung;
Schätzen der Eisenkonzentration in der Probe basierend auf dem detektierten Absorptionsgrad, und somit Bereitstellen eines Analyseergebnisses;
Einstellen der Eisenkonzentration der Probe gemäß dem Analyseergebnis durch:
Speichern einer chemischen Lösung zur Eisenkonzentrationseinstellung und Hinzugeben der chemischen Lösung zur Eisenkonzentrationseinstellung zu einer Lösung in der Zielanlage (170);
Steuern bzw. Regeln der Zugabemenge gemäß dem durch das Analysesystem (40) gewonnen Analyseergebnis.

## Revendications

1. Système de gestion pour une installation de traitement des eaux usées (120, 170) ou une installation de récupération de dioxyde de carbone (140, 170), le système comprenant
un système d'analyse (40) appliqué à la conduite de décharge (132) de l'installation de traitement (120) ; et
un système d'ajustement (160), dans lequel le système d'analyse (40) comprend :
une unité de collecte (41) configurée pour collecter du fer soluble contenu dans un échantillon ;
une unité de réaction (44) configurée pour produire une solution réactionnelle ;
une unité de détection (45) configurée pour détecter une absorbance de la solution réactionnelle ;
une unité de commande d'alimentation (151) configurée pour distribuer le fer soluble collecté dans l'unité de collecte (41) et un réactif dans l'unité de réaction (44),
une unité d'estimation (155) configurée pour estimer la concentration en fer dans l'échantillon sur la base de l'absorbance détectée et ;
dans lequel le système d'ajustement (160) est configuré pour acquérir un résultat d'analyse obtenu par le système d'analyse (40) et est configuré pour ajuster la concentration en fer de l'échantillon conformément au résultat d'analyse ; le système d'ajustement (160) comporte un réservoir (163) qui est configuré pour stocker une solution chimique d'ajustement de concentration en fer et est relié à l'installation cible (170) à travers une conduite d'alimentation (W1), de sorte que la solution chimique d'ajustement de concentration en fer est ajoutée à une solution dans l'installation cible (170) ;
le système d'ajustement (160) comporte un dispositif de commande d'ajout (161) qui est configuré pour commander la quantité de l'ajout conformément au résultat d'analyse obtenu par le système d'analyse (40).

2. Système de gestion selon la revendication 1, dans lequel l'unité de collecte (41) est formée par un extracteur de phase solide utilisant une résine chélatrice.

3. Système de gestion selon la revendication 1 ou 2 comprenant en outre :
un tube d'alimentation en échantillon à travers lequel l'échantillon est distribué dans l'unité de collecte (41) ; et
un tube d'alimentation en éluat à travers lequel un éluat est distribué dans l'unité de collecte (41), dans lequel
l'unité de commande d'alimentation (151) est configurée pour distribuer l'échantillon dans l'unité de collecte (41) à travers le tube d'alimentation en échantillon, puis est configurée pour envoyer l'éluat à l'unité de collecte (41) à travers le tube d'alimentation en éluat, et est configurée pour distribuer l'éluat et le fer soluble dans l'unité de réaction (44).

4. Système de gestion selon la revendication 2 comprenant en outre :
un tube d'alimentation en solution alcaline à travers lequel une solution alcaline est distribuée dans l'unité de collecte (41) ; et
une unité de commande de régénération (152) configurée pour distribuer le fer soluble dans l'unité de réaction (44), puis pour distribuer la solution alcaline dans l'unité de collecte (41).

5. Système de gestion selon l'une quelconque des revendications 1 à 4 comprenant en outre une pompe à seringue (52) configurée pour aspirer du liquide dans un récipient et envoyer une quantité prédéterminée du liquide à partir du récipient, dans lequel
l'unité de commande d'alimentation (151) est configurée pour commander la pompe à seringue (52) afin d'envoyer l'échantillon à l'unité de collecte (41).

6. Système de gestion selon l'une quelconque des revendications 1 à 5 comprenant en outre une pompe (63) configurée pour distribuer de l'air dans l'unité de réaction (44), dans lequel
l'unité de commande d'alimentation (151) est configurée pour amener la pompe (63) à distribuer de l'air dans l'unité de réaction (44), provoquant un bouillonnement d'air pour agiter le fer soluble et le réactif.

7. Système de gestion selon l'une quelconque des revendications 1 à 6 comprenant en outre :
un réservoir d'eau pure (T1) stockant de l'eau pure ;
un tuyau d'alimentation en eau pure à travers lequel l'eau pure est distribuée depuis le réservoir d'eau pure (T1) dans l'une de l'unité de réaction (44) et de l'unité de détection (45) ou les deux ; et
une unité de commande de lavage (153) configurée pour distribuer de l'eau pure dans l'une de l'unité de réaction (44) et de l'unité de détection (45), ou les deux, à travers le tuyau d'alimentation en eau pure.

8. Système de gestion selon l'une quelconque des revendications 1 à 7 comprenant en outre :
un réservoir de solution de référence (T9) stockant une solution de référence dont la concentration en fer est ajustée à l'avance ; et
une unité de commande d'étalonnage (154) configurée pour étalonner l'unité de détection (45) sur la base de la solution réactionnelle obtenue par mélange de la solution de référence et du réactif.

9. Système de gestion selon l'une quelconque des revendications 1 à 8 comprenant en outre un système de notification configuré pour émettre une notification indiquant une anomalie, lorsque le résultat d'analyse se situe en dehors d'une plage de référence de gestion établie à l'avance.

10. Méthode de gestion d'une installation de traitement des eaux usées (120, 170) ou d'une installation de récupération de dioxyde de carbone (140, 170) fonctionnant dans un système d'analyse (40) appliqué à la conduite de décharge (132) de l'installation de traitement (120) comprenant les étapes suivantes :
- la collecte de fer soluble contenu dans un échantillon ;
- la distribution d'un réactif dans l'échantillon pour produire une solution réactionnelle ;
- la détection d'une absorbance de la solution réactionnelle ;
l'estimation de la concentration en fer dans l'échantillon sur la base de l'absorbance détectée fournissant ainsi un résultat d'analyse ;
l'ajustement de la concentration en fer de l'échantillon conformément au résultat d'analyse en :
stockant une solution chimique d'ajustement de concentration en fer et
ajoutant la solution chimique d'ajustement de concentration en fer à une solution dans l'installation cible (170) ;
commandant la quantité de l'ajout conformément au résultat d'analyse obtenu par le système d'analyse (40).
